# EUROPÄISCHE PATENTANMELDUNG

(11) **EP 1 314 718 A1**
(43) Veröffentlichungstag der Anmeldung: **28.05.2003**
(21) Anmeldenummer: 01127912.2
(22) Anmeldetag: 23.11.2001
(51) Int. Cl.: C07C 233/35, C11D 1/62

(54) **Quaternisierte Fettsäureamidoamine**

(71) Anmelder: Cognis Iberia, S.L., 08755 Castellbisbal, (Barcelona) (ES)
(72) Erfinder: Bigorra Llosas, Joaquin, Dr., E-08203 Sabadell (ES); Bonastre Gilabert, Nuria, Dr., E-08210 Barbera del Vall (ES); Sanchez, Agustin, 08921 Santa Coloma De Gramenet (Barc) (ES); Pi Subirana, Rafael, Dr., 08400 Granollers (ES)
(74) Vertreter: Fabry, Bernd, Dr.

(57) **Zusammenfassung**

Vorgeschlagen werden neue kationische Tenside der Formel **(I),** 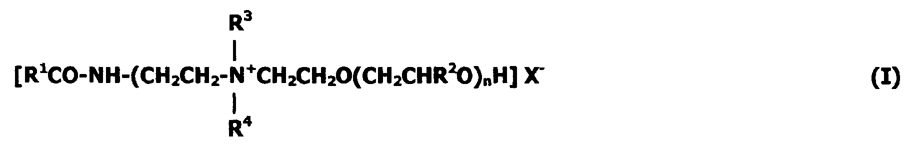 in der R¹CO für einen linearen oder verzweigten, gesättigten oder ungesättigten Acylrest mit 6 bis 22 Kohlenstoffatomen, R² für Wasserstoff oder eine Methylgruppe, R³ für Wasserstoff oder eine Alkylgruppe mit 1 bis 4 Kohlenstoffatomen, R⁴ für eine Gruppe (CH₂CHR²O)ₘH, n und m unabhängig voneinander für Zahlen von 1 bis 50 und X für Halogenid oder Alkylsulfat steht.

## Beschreibung

### Gebiet der Erfindung

Die Erfindung befindet sich auf dem Gebiet der oberflächenaktiven Verbindungen und betrifft neue kationische, Alkylenoxidgruppen enthaltende Tenside, Intermediate zu deren Herstellung, ein Verfahren zu ihrer Herstellung, ihre Verwendung im Bereich der Detergentien und der Kosmetik sowie Mittel, die die Kationtenside zusammen mit Esterquats enthalten.

### Stand der Technik

Kationische Tenside verfügen unter den oberflächenaktiven Stoffen über die einzigartige Fähigkeit, auf feste Oberflächen, wie beispielsweise textile Fasern oder Haare aufzuziehen, so dass es zu Abstoßungseffekten kommt. Im Bereich der Detergentien macht man sich dies bei der Avivage zu Nutze, d.h. dadurch dass sich die Fasern nicht mehr ohne weiteres ineinander verknäueln können, wird den Textilien ein angenehmer Weichgriff verliehen. Im Bereich der Kosmetik erfolgt die Anwendung entsprechend und macht sich durch eine verbesserte Kämmbarkeit bemerkbar. In beiden Fällen wird zudem die statische Aufladung der Fasern verringert. Obschon kationischen Tensiden diese Eigenschaften insgesamt innewohnen, ist der deren Ausprägung natürlich stark von der Struktur abhängig. Auch die übrigen Rezepturbestandteile können einen erheblichen Einfluss auf das Ergebnis haben, so dass es bis heute kaum möglich ist, die Qualität der Effekte vorauszusagen. Tatsächlich beobachtet man nicht selten, dass zwei unterschiedliche Kationtenside mit geringem Leistungsvermögen beträchtliche Synergien entwickeln und im Endeffekt das Niveau leistungsstarker Produkte erreichen oder sogar übertreffen. Von Nachteil beim Einsatz von kationischen Verbindungen ist ihre im Vergleich zu anionischen oder nichtionischen Tensiden geringere biologische Abbaubarkeit. Diese lässt sich im wesentlichen darauf zurückführen, dass kationische Tenside auch über mehr oder weniger biozide Eigenschaften verfügen, was natürlich im Hinblick auf die Mikroorganismen, die diese Stoffe abbauen sollen, vielfach kontraproduktiv ist. Ein typisches Beispiel für eine Gruppe leistungsstarker kationischer Tenside mit Schwächen im biologischen Abbau sind die Quaternierungsprodukte von alkoxylierten Fettaminen.

Die Aufgabe der Erfindung hat somit darin bestanden, neue kationische Tenside zur Verfügung zu stellen, die aus anwendungstechnischer Sicht mit Quaternierungsprodukten alkoxylierter Fettamine wenigstens vergleichbar sind, jedoch über eine deutlich bessere biologische Abbaubarkeit und antistatische Wirksamkeit verfügen.

### Beschreibung der Erfindung

Gegenstand der Erfindung sind neue kationische Tenside der Formel (I),

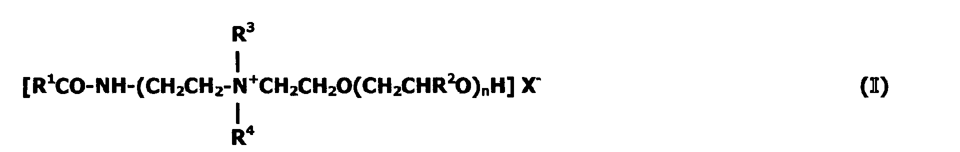

in der R¹CO für einen linearen oder verzweigten, gesättigten oder ungesättigten Acylrest mit 6 bis 22 Kohlenstoffatomen, R² für Wasserstoff oder eine Methylgruppe, R³ für Wasserstoff oder eine Alkylgruppe mit 1 bis 4 Kohlenstoffatomen, R⁴ für eine Gruppe (CH₂CHR²O)ₘH, n und m unabhängig voneinander für Zahlen von 1 bis 50 und X für Halogenid oder Alkylsulfat steht.

Die neuen kationischen Tenside erfüllen das geforderte Leistungsprofil in ausgezeichneter Weise. Ein Produkt auf Basis Kokosfettsäure + Aminoethylethanolamin, ethoxyliert mit 10 Mol Ethylenoxid und quaterniert mit Dimethylsulfat zeigte gegenüber einem Vergleichsprodukt (Kokosamin+10 EO, quaterniert mit Dimethylsulfat) die gleiche Avivage, jedoch eine um 50 % verbesserte biologische Abbaubarkeit (STURM-Test). In gleicher Weise wird eine deutliche Verbesserung der Ausrüstung gegen statische Aufladung beobachtet.

Ein weiterer Gegenstand der Erfindung betrifft nichtionische Tenside der Formel (II), 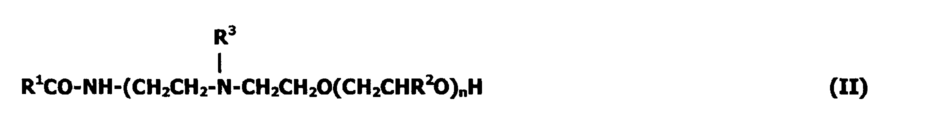 in der R¹CO für einen linearen oder verzweigten, gesättigten oder ungesättigten Acylrest mit 6 bis 22 Kohlenstoffatomen, R² für Wasserstoff oder eine Methylgruppe, R³ für Wasserstoff oder eine Alkylgruppe mit 1 bis 4 Kohlenstoffatomen und steht. Bei den Stoffen der Formel **(II)** handelt es sich um Intermediate zur Herstellung der Kationtenside der Formel **(I).**

Ein dritter Gegenstand der Erfindung betrifft ein Verfahren zur Herstellung kationischer Tenside der Formel **(I),** 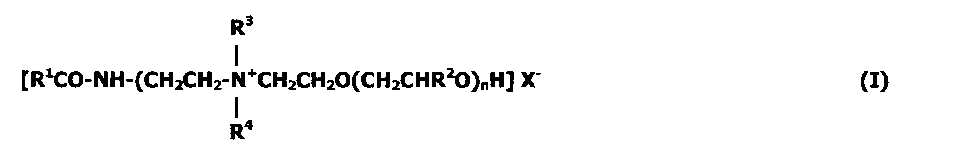 in der R¹CO für einen linearen oder verzweigten, gesättigten oder ungesättigten Acylrest mit 6 bis 22 Kohlenstoffatomen, R² für Wasserstoff oder eine Methylgruppe, R³ für Wasserstoff oder eine Alkylgruppe mit 1 bis 4 Kohlenstoffatomen, R⁴ für eine Gruppe (CH₂CHR²O)ₘH, n und m unabhängig voneinander für Zahlen von 1 bis 50 und X für Halogenid oder Alkylsulfat steht, bei dem man
(a) Fettsäuren der Formel **(III),**

   **R**^{**1**}**CO-OH** (III)

   mit Diaminen der Formel **(IV)** kondensiert,

   **H**_{**2**}**NCH**_{**2**}**CN**_{**2**}**NR**^{**3**}**CH**_{**2**}**CH**_{**2**}**OH** (IV)
(b) die resultierenden Imidazoline mit Wasser hydrolysiert,
(c) die dabei entstandenen Fettsäureamidoamine mit Alkylenoxiden umsetzt, und schließlich
(d) die so erhaltenen Alkoxylierungsprodukte in an sich bekannter Weise quaterniert.

### Kondensation und Hydrolyse

Als Ausgangsstoffe für die erfindungsgemäßen neuen Kationtenside kommen vorzugsweise solche Fettsäuren der Formel **(III)** in Betracht, bei der R¹CO für einen linearen, gesättigten Acylrest mit 12 bis 18 Kohlenstoffatomen steht. Typische Beispiele sind Laurinsäure, Myristinsäure, Palmitinsäure und Stearinsäure sowie deren technische Gemische. Besonders bevorzugt ist der Einsatz von Kokosfettsäure. Unter den Diaminen, die mit den Fettsäuren unter Imidazolinbildung kondensiert werden, kommt die höchste Bedeutung dem Aminoethylethanolamin zu. Die Kondensation kann in an sich bekannter Weise durchgeführt werden, d.h. mit überstöchiometrischer Einsatzmenge der Fettsäure als der billigeren Komponente, kontinuierlicher Entfernung des Kondensationswassers aus der Reaktionsmischung sowie Abtrennung der nicht umgesetzten Fettsäuren. Die nachfolgende Hydrolyse unter Öffnung des Imidazolinringes erfolgt vorzugsweise mit einem mindestens 50 mol%igen Überschuss an Wasser bei Temperaturen im Bereich von 50 bis 30 und vorzugsweise bei 80 bis 120 °C und gegebenenfalls unter Druck, um die Bildung linearer Hydrolyseprodukte vom nachfolgenden Typ

RCO-NHCH₂CH₂NHCH₂CH₂OH

zu begünstigen. Die Hydrolyse kann auch unter alkalischen Bedingungen stattfinden.

### Alkoxylierung

Die Alkoxylierung der intermediär entstandenen Fettsäureamidoamine kann ebenfalls in an sich bekannter Weise erfolgen. Da die Reaktionsmischung ohnehin basisch reagiert, ist die Verwendung eines Katalysators nicht zwingend erforderlich. In der Regel erfolgt die Anlagerung der Alkylenoxide - sei es blockweise oder randomisiert - bei Temperaturen im Bereich von 100 bis 140 °C unter einem autogenen Druck von 1,1 bis 5 bar. Üblicherweise werden durchschnittlich 1 bis 50 und vorzugsweise 5 bis 20 Mol Ethylenoxid und/oder Propylenoxid, vorzugsweise 5 bis 10 Mol Ethylenoxid oder 1 bis 2 Mol Propylenoxid und anschließend 5 bis 15 Mol Ethylenoxid angelagert.

### Quaternierung

Schließlich kann auch die Quaternierung in an sich bekannter Weise erfolgen, d.h. die zuvor hergestellten Alkoxylate und die Quaternierungsmittel werden in praktisch stöchiometrischen Mengen bei Temperaturen unterhalb von 70, vorzugsweise 50 bis 65 °C zur Reaktion gebracht. Um die Anwesenheit von Spuren des Alkylierungsmittels auszuschließen und sich eine teure Aufarbeitung zu sparen, empfiehlt es sich, auf eine vollständige Quaternierung zu verzichten und statt dessen das Quaternierungsmittel in geringem Unterschuss zu verwenden. Vorzugsweise handelt es sich hierbei um Methylchlorid oder insbesondere Dimethylsulfat.

### Gewerbliche Anwendbarkeit

Die neuen kationischen Tenside verleihen sowohl natürlichen als synthetischen Fasern einen angenehmen Weichgriff und vermindern die statische Aufladung. Kürzerkettige Verbindungen besitzen zudem biozide Aktivität. Ein weiteres Einsatzgebiet ist die Reinigung harter Oberflächen, beispielsweise im Bereich der automatischen Hochdruckreinigung von Fahrzeugen, bei der die Verminderung der statischen Aufladung ebenfalls von besonderer Bedeutung ist. Weitere Gegenstände der Erfindung betreffen daher ihre Verwendung zur Herstellung von Textilnachbehandlungsmitteln, speziell Weichspülern, von kosmetischen Zubereitungen, speziell solchen zur Reinigung und Pflege der Haare, sowie von Mitteln zur Reinigung harter Oberflächen, speziell solchen zur Hochdruckreinigung von Fahrzeugen, in denen sie in Mengen von 01, bis 30, vorzugsweise 1 bis 15 und insbesondere 2 bis 10 Gew.-% - bezogen auf die Mittel - enthalten sein können.

### Mischungen mit Esterquats

In einer weiteren bevorzugten Ausführungsform der Erfindung können die neuen kationischen Tenside mit quartären Verbindungen vom Esterquat-Typ abgemischt werden, wobei das Gewichtsverhältnis im Bereich von 90 : 10 bis 10 : 90, vorzugsweise bei 75 : 25 bis 25 : 75 und insbesondere 60 : 40 bis 40 : 60 liegen kann. Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE 4308794 C1** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am. Oil.Chem.Soc., 71, 97 (1994)** sowie I.Shapiro in **Cosm.Toil. 109, 77 (1994)** erschienen.

Die quaternierten Fettsäuretriethanolaminestersalze folgen beispielsweise der Formel **(V),**

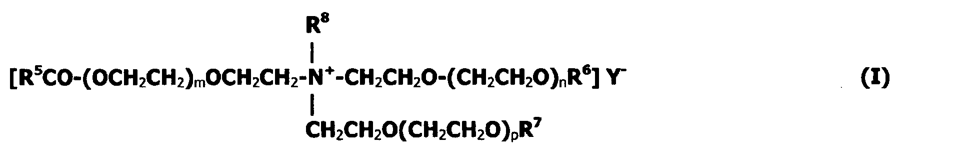

in der R⁵CO für einen Acylrest mit 6 bis 22 Kohlenstoffatomen, R⁶ und R⁷ unabhängig voneinander für Wasserstoff oder R⁵CO, R⁸ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und Y für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab. Aus anwendungstechnischer Sicht haben sich quaternierte Fettsäuretriethanolaminestersalze der Formel **(I)** als besonders vorteilhaft erwiesen, in der R⁵CO für einen Acylrest mit 16 bis 18 Kohlenstoffatomen, R⁶ für R⁵CO, R⁷ für Wasserstoff, R⁸ für eine Methylgruppe, m, n und p für 0 und Y für Methylsulfat steht. Entsprechende Produkte sind unter der Marke Dehyquart® AU (Cognis Deutschland GmbH) im Handel.

Neben den quaternierten Fettsäuretriethanolaminestersalzen kommen als Esterquats ferner auch quaternierte Estersalze von Fettsäuren mit Diethanolalkylaminen der Formel (VI) in Betracht,

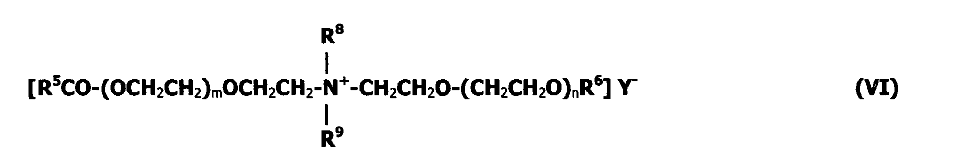

in der R⁵CO für einen Acylrest mit 6 bis 22 Kohlenstoffatomen, R⁶ für Wasserstoff oder R⁵CO, R⁸ und R⁹ unabhängig voneinander für Alkylreste mit 1 bis 4 Kohlenstoffatomen, m und n in Summe für 0 oder Zahlen von 1 bis 12 und Y für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel (**VII**) zu nennen,

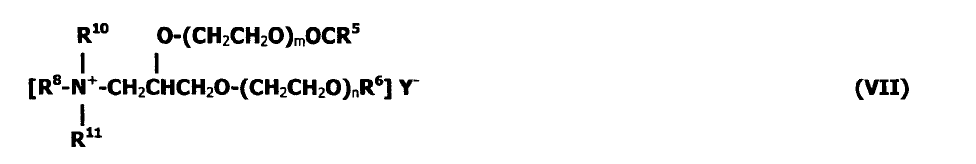

in der R⁵CO für einen Acylrest mit 6 bis 22 Kohlenstoffatomen, R⁶ für Wasserstoff oder R⁵CO, R⁸, R¹⁰ und R¹¹ unabhängig voneinander für Alkylreste mit 1 bis 4 Kohlenstoffatomen, m und n in Summe für 0 oder Zahlen von 1 bis 12 und Y für Halogenid, Alkylsulfat oder Alkylphosphat steht.

Des weiteren kommen als Esterquats noch Stoffe in Frage, bei denen die Ester- durch eine Amidbindung ersetzt ist und die vorzugsweise basierend auf Diethylentriamin der Formel (**VIII**) folgen,

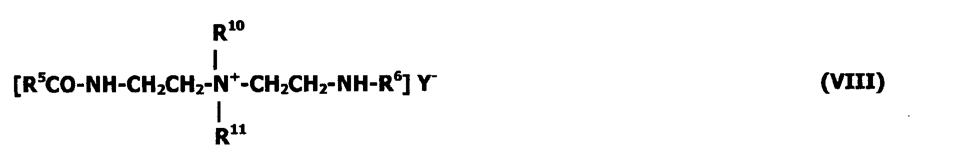

in der R⁵CO für einen Acylrest mit 6 bis 22 Kohlenstoffatomen, R⁶ für Wasserstoff oder R⁵CO, R¹⁰ und R¹¹ unabhängig voneinander für Alkylreste mit 1 bis 4 Kohlenstoffatomen und Y für Halogenid, Alkylsulfat oder Alkylphosphat steht. Derartige Amidesterquats sind beispielsweise unter der Marke Incroquat® (Croda) im Markt erhältlich.

Schließlich kommen als Esterquats auch Stoffe in Frage, die auf Basis von ethoxyliertem Ricinusöl oder dessen Härtungsprodukten erhältlich sind und vorzugsweise der Formel **(IX)** folgen,

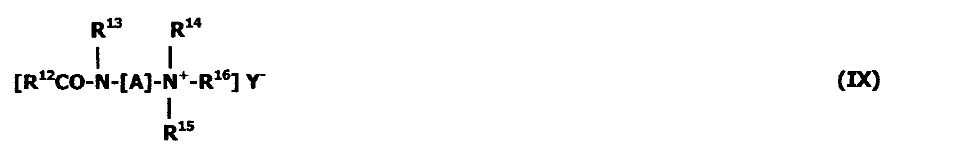

in der R¹²CO für einen gesättigten und/oder ungesättigten ethoxylierten Hydroxyacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R¹³, R¹⁴ und R¹⁵ unabhängig voneinander für Wasserstoff oder eine Alkylgruppe mit 1 bis 4 Kohlenstoffatomen, R¹⁶ für einen Alkylrest mit 1 bis 4 Kohlenstoffatomen oder einen Benzylrest und Y für Halogen, Alkylsulfat oder Alkylphosphat steht.

Hinsichtlich der Auswahl der bevorzugten Fettsäuren und des optimalen Veresterungsgrades gelten die für **(V)** genannten Beispiele auch für die Esterquats der Formeln **(VI)** bis **(IX).**

Zur Herstellung der Esterquats der Formeln (V) bis (IX) kann sowohl von Fettsäuren als auch den entsprechenden Triglyceriden ausgegangen werden. Ein solches Verfahren, das stellvertretend für den entsprechenden Stand der Technik genannt werden soll, wird in der europäischen Patentschrift **EP 0750606 B1** (Cognis) vorgeschlagen. Ebenfalls ist es möglich, die Kondensation der Alkanolamine mit den Fettsäuren in Gegenwart definierter Mengen an Dicarbonsäuren, wie z.B. Oxalsäure, Malonsäure, Bernsteinsäure, Maleinsäure, Fumarsäure, Glutarsäure, Adipinsäure, Sorbinsäure, Pimelinsäure, Azelainsäure, Sebacinsäure und/oder Dodecandisäure durchzuführen. Auf diese Weise kommt es zur einer partiell oligomeren Struktur der Esterquats, was sich insbesondere bei Mitverwendung von Adipinsäure auf die Klarlöslichkeit der Produkte vorteilhaft auswirken kann. Entsprechende Produkte unter der Marke Dehyquart® D 6003 (Cognis Deutschland GmbH) sind im Handel erhältlich und werden beispielsweise in der Europäischen Patentschrift **EP 0770594** B1 (Cognis) beschrieben. Üblicherweise gelangen die Esterquats in Form 50 bis 90 Gew.-%iger alkoholischer Lösungen in den Handel, die bei Bedarf problemlos mit Wasser verdünnt werden können.

### Beispiele

**Beispiel 1.** In einem Druckreaktor wurden bei 75 °C 500 g (1,74 Mol) eines Imidazolins auf Basis von Kokosfettsäure und Aminoethylethanolamin und 50 g (2,8 Mol) Wasser vorgelegt und dann 2 h auf 110 °C erwärmt. Der Reaktor wurde zunächst mit Stickstoff gespült und dann portionsweise 845 g (19,2 Mol) Ethylenoxid aufgegeben, wobei der Druck bis auf 3,5 bar anstieg. Nach Beendigung der Reaktion wurde der Reaktor abgekühlt und entspannt. 675 g (0,99 Mol) des so erhaltenen Ethoxylierungsproduktes wurden in einen zweiten Reaktor überführt und bei 65 °C portionsweise mit 118 g (0,94 Mol) Dimethylsulfat versetzt, wobei die Temperatur unter 70 °C gehalten wurde. Anschließend wurde die Reaktionsmischung 4 h bei 65 °C gerührt. Das Quaternierungsprodukt wurde als transparente, rötlich gefärbte Flüssigkeit erhalten.

## Patentansprüche

1. Kationische Tenside der Formel **(I),** 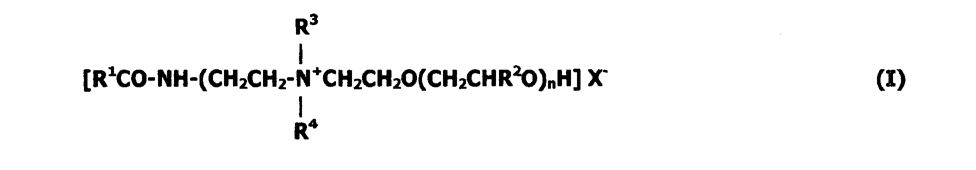 in der R¹CO für einen linearen oder verzweigten, gesättigten oder ungesättigten Acylrest mit 6 bis 22 Kohlenstoffatomen, R² für Wasserstoff oder eine Methylgruppe, R³ für Wasserstoff oder eine Alkylgruppe mit 1 bis 4 Kohlenstoffatomen, R⁴ für eine Gruppe (CH₂CHR²O)ₘH, n und m unabhängig voneinander für Zahlen von 1 bis 50 und X für Halogenid oder Alkylsulfat steht.

2. Nichtionische Tenside der Formel (**II**), 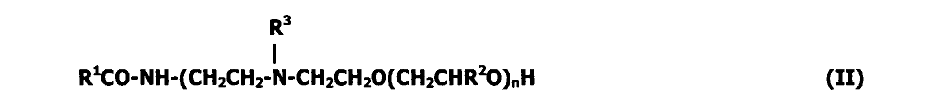 in der R¹CO für einen linearen oder verzweigten, gesättigten oder ungesättigten Acylrest mit 6 bis 22 Kohlenstoffatomen, R² für Wasserstoff oder eine Methylgruppe, R³ für Wasserstoff oder eine Alkylgruppe mit 1 bis 4 Kohlenstoffatomen und steht.

3. Verfahren zur Herstellung kationischer Tenside der Formel **(I),** 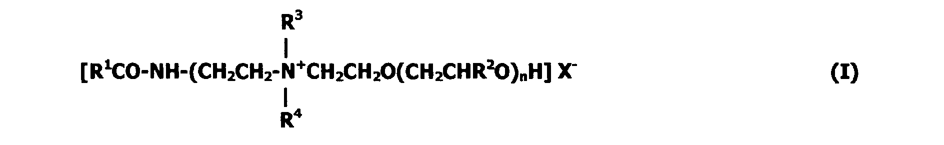 in der R¹CO für einen linearen oder verzweigten, gesättigten oder ungesättigten Acylrest mit 6 bis 22 Kohlenstoffatomen, R² für Wasserstoff oder eine Methylgruppe, R³ für Wasserstoff oder eine Alkylgruppe mit 1 bis 4 Kohlenstoffatomen, R⁴ für eine Gruppe (CH₂CHR²O)ₘH, n und m unabhängig voneinander für Zahlen von 1 bis 50 und X für Halogenid oder Alkylsulfat steht, bei dem man
(a) Fettsäuren der Formel **(III),**
**R**^{**1**}**CO-OH** (III)
mit Diaminen der Formel **(IV)** kondensiert,
**H**_{**2**}**NCH**_{**2**}**CH**_{**2**}**NR**^{**3**}**CH**_{**2**}**CH**_{**2**}**OH** (III)
(b) die resultierenden Imidazoline mit Wasser hydrolysiert,
(c) die dabei entstandenen Fettsäureamidoamine mit Alkylenoxiden umsetzt, und schließlich
(d) die so erhaltenen Alkoxylierungsprodukte in an sich bekannter Weise quaterniert.

4. Verfahren nach Anspruch 3, **dadurch gekennzeichnet, dass** man Fettsäuren der Formel (I) einsetzt, bei der R¹CO für einen linearen, gesättigten Acylrest mit 12 bis 18 Kohlenstoffatomen steht.

5. Verfahren nach den Ansprüchen 3 und 4, **dadurch gekennzeichnet, dass** man als Fettsäure Kokosfettsäure einsetzt

6. Verfahren nach mindestens einem der Ansprüche 3 bis 5, **dadurch gekennzeichnet, dass** man als Diamin Aminoethylethanolamin einsetzt.

7. Verfahren nach mindestens einem der Ansprüche 3 bis 6, **dadurch gekennzeichnet, dass** man die Imidazoline mit einem mindestens 50 mol%-igen Überschuss an Wasser hydrolysiert.

8. Verfahren nach mindestens einem der Ansprüche 3 bis 7, **dadurch gekennzeichnet, dass** man die Fettsäureamidoamine mit durchschnittlich 1 bis 50 Mol Ethylenoxid und/oder Propylenoxid umsetzt.

9. Verfahren nach mindestens einem der Ansprüche 3 bis 8, **dadurch gekennzeichnet, dass** man die ethoxylierten Fettsäureamidoamine mit Dimethylsulfat oder Methylchlorid quaterniert.

10. Verwendung von kationischen Tensiden nach Anspruch 1 zur Herstellung von Wäschenachbehandlungsmitteln.

11. Verwendung von kationischen Tensiden nach Anspruch 1 zur Herstellung von kosmetischen Zubereitungen.

12. Verwendung von kationischen Tensiden nach Anspruch 1 zur Herstellung von Mitteln zur Reinigung harter Oberflächen.

13. Mittel, enthaltend
(a) kationische Tenside nach Anspruch 1 und
(b) Esterquats.

14. Mittel nach Anspruch 13, **dadurch gekennzeichnet, dass** sie die Komponenten (a) und (b) im Gewichtsverhältnis 90 : 10 bis 10 : 90 enthalten.
